# EUROPEAN PATENT SPECIFICATION

(11) **EP 4 352 036 B1**
(45) Date of publication and mention of the grant of the patent: **25.12.2024**
(21) Application number: 22733360.6
(22) Date of filing: 09.06.2022
(51) Int. Cl.: C07C 51/42, C07C 51/43, C07C 55/06, C07C 59/153

(54) **PROCESS AND SYSTEM FOR THE RECOVERY OF GLYOXYLIC ACID**
VERFAHREN UND SYSTEM ZUR GEWINNUNG VON GLYOXYLSÄURE
PROCÉDÉ ET SYSTÈME DE RÉCUPÉRATION D'ACIDE GLYOXYLIQUE

(30) Priority: 09.06.2021 EP 21178546
(43) Date of publication of application: 17.04.2024
(73) Proprietor: Avantium Knowledge Centre B.V., 1014 BV Amsterdam (NL)
(72) Inventor: KRASOVIC, Julia, 1014 BV Amsterdam (NL); JONGERIUS, Anna Louise, 1014 BV Amsterdam (NL); ESMAEILI, Faezeh, 1014 BV Amsterdam (NL)
(74) Representative: Avantium Intellectual Property
(86) International application number: PCT/EP2022/065630
(87) International publication number: WO 2022/258732

(56) References cited:
- CN-A- 109 852 987
- US-A1- 2015 344 397

## Description

### Field

The present invention relates to a process and a system for the recovery and purification of glyoxylic acid from a process stream comprising glyoxylic acid and oxalic acid.

### Background art

Glyoxylic acid is an important reagent in the preparation of industrially relevant compounds, including pharmaceuticals such as β-lactam antibiotics (methicillin, oxacillin, and nafcillin) and allantoin, as well as vanillin which is perfume and food products.

Glyoxylic acid (COOHCHO) can be prepared either by controlled oxidation of glyoxal or by electrochemical reduction of oxalic acid (COOHCOOH). The electrochemical (electrolytic) reduction of oxalic acid to give glyoxylic acid has been known for more than 100 years and is generally carried out in an aqueous, acidic medium, at low temperature, on electrodes having a high hydrogen overpotential, such as lead-based electrodes. To avoid electrooxidation of both oxalic acid and glyoxylic acid, the process is typically carried out in a divided cell using ion exchange membrane and as catholyte a solution of oxalic acid (see e.g., F. Goodridge et al., Journal of Applied Electrochemistry 10 (1980) pp. 55-60; J.R. Ochoa et al., Journal of Applied Electrochemistry 23 (1993) 905-909).

The main reaction on the cathode surface is

COOHCOOH + 2H⁺ + 2e⁻ → COOHCHO + H₂O

The main reaction on the anode surface is

2 H₂O → O₂ + 4 H⁺ + 4e⁻

The aqueous product stream in prior art processes for the electrochemical conversion of oxalic acid to glyoxylic acid typically contains up to 10 wt% glyoxylic acid and a smaller amount of oxalic acid. To recover the glyoxylic acid product, the aqueous product stream is generally subjected to a combination of separation and concentration steps in order to separate glyoxylic acid from oxalic acid and to remove water, including crystallization and filtration of the oxalic acid and evaporation of water, optionally under vacuum. Another method for separating glyoxylic acid from oxalic acid is the selectively precipitation and subsequent separation of oxalic acid and glyoxylic acid as their barium or calcium salts under specific pH conditions as described in US3998878 A. A drawback of this method is that additional steps are required to recover glyoxylic acid from its salt form. US4902828 describes a chemical extraction method for the recovery of glyoxylic acid from aqueous solutions containing several acids, wherein the aqueous solution is mixed with an organic nitrogen compound at s temperature as high as 50 °C, the phases are separated, and the glyoxylic acid is extracted from the organic phase with water, at a higher temperature. This method, too, requires the use of additional chemicals that need to be regenerated and recycled back to the process. Another method for the separation of water from glyoxylic acid is vacuum distillation such as described for example in CN102060690B. Herein, by applying reduced pressure and a stepwise increase of the temperature, glyoxylic acid is concentrated to 85 to 90 percent. A drawback of such a vacuum distillation process is its high energy requirement and the need for large distillation towers.

Recently, improved processes have been developed by the applicant that result in an increased concentration of glyoxylic acid in the product stream. For example, in European patent application 20210026.9 an electrochemical reduction process of oxalic acid (OA) to glyoxylic acid (GA) is disclosed, wherein additional oxalic acid is supplied to the process in concentrated form and wherein the oxalic acid concentration in the catholyte is kept between pre-defined limits throughout the process, resulting in a concentration of glyoxylic acid in the product stream well exceeding 10 wt%. However, even in such improved processes, oxalic acid and water must be removed to obtain glyoxylic acid that has the desired degree of purity, for example 99% pure glyoxylic acid monohydrate.

CN 109 852 987 discloses a process wherein oxalic acid is cooled and crystallized. The remaining solution is neutralized and concentrated using reverse osmosis, thus obtaining a sodium oxalate precipitate and a concentrated sodium glyoxylate solution.

Hence, there is a need in the art for a process for the recovery and purification of glyoxylic acid from an aqueous stream comprising glyoxylic acid and oxalic acid, in particular an aqueous product stream comprising glyoxylic acid and oxalic acid originating from the electrochemical reduction of oxalic acid (OA) to glyoxylic acid (GA).

### Summary

Accordingly, in a first aspect there is provided a process for the recovery of glyoxylic acid from an aqueous solution comprising glyoxylic acid and oxalic acid, said process comprising the steps of:
(i) providing an aqueous solution comprising glyoxylic acid and oxalic acid
(ii) precipitating at least a portion of the oxalic acid using low-temperature crystallization at a temperature of at most 18 °C,
(iii) separating the precipitated oxalic acid from the solution to obtain a solution depleted in oxalic acid,
(iv) concentrating the solution depleted in oxalic obtained in step (iii) using reverse osmosis,
repeating steps (ii)-(iv) with the concentrated solution obtained in step (iv) until a purified glyoxylic acid stream with a predetermined degree of purity is obtained.

By using sequentially alternating crystallization and membrane filtration steps, in the present process glyoxylic acid present in an aqueous solution also comprising oxalic acid, such as an aqueous product stream resulting from the electrochemical conversion of oxalic acid, is both purified and concentrated. More specifically, in each cycle oxalic acid is precipitated and separated, and the resulting oxalic acid-depleted solution is concentrated, which in turn facilitates precipitation of oxalic acid in a subsequent crystallization step. These alternating crystallization-concentration cycles are continued until sufficient water and oxalic acid have been removed to reach the desired concentration and degree of purity of glyoxylic acid.

The amount of oxalic acid that precipitates and can be recovered can be controlled by adjusting the temperature in the crystallisation unit. For example, at a temperature in the range of 0°C -5°C, around 70-80 wt. % of the oxalic acid present in the initial solution can be separated from the solution.

The process of the present invention does not require the use of additional reagents or solvents and allows adjusting the recovery percentage of both glyoxylic acid and oxalic acid by controlling the crystallization and concentration conditions and/or the number of crystallization-concentration cycles. Compared to other techniques known in the art for the recovery of glyoxylic acid, the energy costs for the present process are reduced to the sequential nature of the process, the absence of high-temperature separation techniques and the absence of additional chemical compounds or solvents in the process. Advantageously, the oxalic acid recovered in the crystallization steps is collected and recycled to the electrolysis process.

The present process is particularly suitable for recovering and purifying glyoxylic acid from an aqueous process stream resulting from the electrochemical reduction of oxalic acid using a process improved by the present applicant, wherein the aqueous process stream comprises an increased concentration of glyoxylic acid and oxalic acid compared to prior art electroreduction processes.

In a further aspect, the invention relates to a reaction system for carrying out the process according to any one of the preceding claims, said system comprising a plurality of sequentially and alternatingly connected crystallisation units and reverse osmosis units,
wherein an outlet of each crystallisation unit is fluidly connected with an inlet of a reverse osmosis unit following it in sequential order, and
wherein the first crystallisation unit is configured to receive an aqueous feed stream comprising glyoxylic acid and oxalic acid, and
wherein each subsequent crystallisation unit comprises an inlet fluidly connected with an outlet of a reverse osmosis unit preceding it in sequential order.

### Brief description of the drawings

Fig. 1 is a schematic diagram of the process of the invention.
Fig. 2 is a schematic diagram of the process of the invention.

### Detailed description

The present invention provides a process for recovering and purifying glyoxylic acid from an aqueous solution comprising glyoxylic acid and oxalic acid.

This aqueous solution comprising glyoxylic acid and oxalic acid may be obtained in an electrochemical reduction process wherein oxalic acid is reduced to glyoxylic acid in the cathode compartment of an electrochemical cell. The electrochemical reduction of oxalic acid to glyoxylic acid is well known in the art and generally involves providing an electrochemical cell comprising a cathode compartment comprising a cathode, a separating membrane, an anode compartment and an anode, wherein the cathode compartment comprise a catholyte comprising an aqueous solution of oxalic acid, and an electric potential difference between the cathode and the anode sufficient to reduce oxalic acid to glyoxylic acid in the cathode compartment. A cathodic product stream comprising an aqueous solution of glyoxylic acid and unreacted oxalic acid can be withdrawn from the cathode compartment.

The aqueous solution of glyoxylic acid and oxalic acid is provided as a feed for a process for recovering and purifying glyoxylic acid, wherein the aqueous solution of glyoxylic acid and oxalic acid is subjected to a series of crystallization-concentration cycles.

The aqueous solution of glyoxylic acid and oxalic acid provided as a feed to the first crystallization unit typically comprises glyoxylic acid in a concentration of 5-20 wt%, preferably 8-18 wt%, more preferably 10-16 wt%, most preferably 11-13 wt%. The aqueous solution of glyoxylic acid and oxalic acid provided as a feed to the first crystallization unit typically comprises oxalic acid in a concentration of 3-12 wt%, preferably 5-10 wt%, more preferably 6-8 wt%.

In one embodiment, the process of the present disclosure is utilized for recovering and purifying glyoxylic acid from an aqueous process stream resulting from the electrochemical reduction of oxalic acid using a process improved by the present applicant, wherein the aqueous process stream comprises an increased concentration of glyoxylic acid and oxalic acid due to the maintenance of constantly high oxalic acid levels by the addition of concentrated oxalic acid to the catholyte during the electrochemical conversion of oxalic acid to glyoxylic acid. Such a process is disclosed in European patent application 20210026.9, and allows for product streams having a glyoxylic acid concentration in excess of 15 wt%.

Oxalic acid has a low solubility in water compared to glyoxylic acid. By reducing the temperature in the crystallisation unit, selective precipitation of the oxalic acid can be established. The precipitated oxalic acid is separated from the aqueous solution by means of e.g., filtration, sedimentation or centrifugation, preferably filtration, resulting in a solution of glyoxylic acid that is depleted of oxalic acid.

Depending on the conditions in the crystallization unit, such as the cooling temperature, a smaller or larger fraction of the oxalic acid present in the aqueous feed stream comprising oxalic acid and glyoxylic acid is precipitated and subsequently separated from the solution. Said crystallization unit may be any crystallizing device capable of selectively crystallizing at least a portion of the oxalic acid present in the feed stream. It may for example be a suspension, such as a standard tank freezer, a suspension crystallizer, a parallel crystallizer, or a layer crystallization device.

As used herein, "low-temperature crystallization" means crystallization at a temperature of at most 18 °C, preferably at most 15 °C. The temperature in the in the crystallization step is typically in the range of -10 to 15 °C. Preferably, the temperature in the in the crystallization step is in the range of -8 to 10 °C, more preferably in the range of -5 to 5 °C.

Typically, in the crystallization step at least 40 wt%, preferably at least 50 wt%, more preferably at least 70 wt%, most preferably at least 80 wt% of the oxalic acid present in the feed stream is precipitated and separated from the feed stream. Generally, no glyoxylic acid is co-precipitated in the crystallization step.

The oxalic acid-depleted solution containing glyoxylic acid resulting from the preceding crystallization step is supplied as a feed to a reverse osmosis (RO) unit. In the reverse osmosis (RO) unit, a reverse osmosis (RO) membrane is used to separate the water from the organic compounds, thus increasing the concentration of glyoxylic acid and any remaining oxalic acid in the solution. Reverse osmosis is a well-known separation and purification process that uses a partially permeable membrane to separate molecules, ions and particles from water by using an applied pressure to overcome the osmotic pressure. The separation mechanism is based on differences in solubility or diffusivity of the compounds, and the process is dependent on pressure, solute concentration, and other conditions. In the reverse osmosis step of the present disclosure, any suitable reverse osmosis (RO) unit can be used that has a membrane that is capable of separating the glyoxylic acid and any remaining oxalic acid in the solution obtained from the preceding oxalic acid crystallization step. Examples of such membranes are dedicated RO membranes, typically comprising a composite structure of a semi-permeable thin film of polyamide deposited on top of an e.g. polyether sulfone or polysulfone porous layer on top of a non-woven fabric support sheet. In the reverse osmosis (RO) unit, a reverse osmosis (RO) membrane is used to separate the water from the organic compounds, thus increasing the concentration of glyoxylic acid and any remaining oxalic acid in the solution. Typically, at least 50 wt%, preferably at least 60 wt%, preferably at least 70 wt%, most preferably at least 80 wt%, of water is removed from the feed stream, resulting in a concentrated aqueous solution of glyoxylic acid and optionally minor amounts of oxalic acid.

In one embodiment, the concentration of glyoxylic acid after the first reverse osmosis dehydration step is 8 to 45 wt%, preferably 20 to 40 wt%, more preferably 33 to 36 wt%.

The concentrated solution may be sent to the next crystallization unit to further separate and remove oxalic acid, whereafter the solution further depleted of oxalic acid is supplied to a subsequent reverse osmosis unit. As a result, the glyoxylic acid concentration will increase with each step while the oxalic acid is removed every cycle. These alternating precipitation/separation and concentration (crystallization-reverse osmosis) cycles are continued until sufficient water and oxalic acid have been removed to reach the desired purity of glyoxylic acid.

In one embodiment, the process involves at least one crystallization-reverse osmosis cycle. In a preferred embodiment, the process involves at least two, preferably at least three, more preferably at least four consecutive crystallization-reverse osmosis cycles. The conditions maintained in the separate, consecutive crystallization-reverse osmosis cycles may be the same, of may differ between two or more the cycles. For example, a lower crystallization temperature may be employed in a second crystallization step as compared to the first crystallization step, and different pressures may be used in the different reverse osmosis steps.

The precipitation/separation and concentration cycles are repeated until a purified glyoxylic acid stream with a desired degree of purity is obtained. Preferably, the stream resulting from the final concentration step comprises glyoxylic acid in a concentration at least 40 wt%, more preferably at least 45 wt%, most preferably around 50 wt%. In one embodiment, this concentrated glyoxylic acid monohydrate is further purified to glyoxylic acid monohydrate with a degree of purity of at least 90 wt%, more preferably at least 95 wt%, most preferably at least 99 wt%. In one embodiment, vacuum distillation is used for this further purification step.

In one embodiment, at least a portion the oxalic acid separated in step (iii) is provided to an electrochemical process for the conversion of oxalic acid to glyoxylic acid. In such case, the recycle stream containing oxalic acid is provided as a feed to the cathode department of an electrochemical cell configured for the reduction of oxalic acid to glyoxylic acid. This is advantageous compared to prior art processes in which the precipitated product is typically destroyed. Depending on the number of crystallization steps in the process, in each step or in a selected number of steps the oxalic acid separated may be recycled as a feed stream for the oxalic acid electrochemical conversion process. In one embodiment, substantially all, or all of the oxalic acid separated from the solution in the crystallization step is recycled as a feed to an electrochemical process for the conversion of oxalic acid to glyoxylic acid.

In another aspect of the present disclosure there is provided a system for carrying out the process as described herein, said system comprising a plurality of sequentially and alternatingly connected crystallisation units and reverse osmosis units,
wherein an outlet of each crystallisation unit is fluidly connected with an inlet of a reverse osmosis unit following it in sequential order,
wherein the first crystallisation unit is configured to receive an aqueous feed stream comprising glyoxylic acid and oxalic acid, and
wherein each subsequent crystallisation unit comprises an inlet fluidly connected with an outlet of a reverse osmosis unit preceding it in sequential order.

In this system, the outlet of a first crystallization unit is fluidly connected with the inlet of a first reverse osmosis unit, and the outlet of said first reverse osmosis unit is fluidly connected to the inlet of a second crystallization unit arranged downstream of said first reverse osmosis unit. The outlet of said second crystallization unit is fluidly connected to the inlet of a second reverse osmosis unit located downstream of the second crystallization unit. The outlet of the second reverse osmosis unit system may optionally be fluidly connected to the inlet of a third crystallization unit arranged downstream of said second reverse osmosis unit, and the outlet of said third crystallization unit is fluidly connected to the inlet of a third reverse osmosis unit located downstream of the second crystallization unit. Optionally, fourth, fifth and so on alternatingly connected crystallisation units and reverse osmosis units are fluidly connected to and located downstream of the outlet of the third reverse osmosis unit in the same manner as described above.

Preferably, the system comprises at least two crystallisation units and at least two reverse osmosis units connected sequentially and alternatingly. In one embodiment, the system comprises at least three crystallisation units and at least three reverse osmosis units connected alternatingly and sequentially. In another embodiment, the system comprises at least four, preferably at least five or six crystallisation units and the same number of reverse osmosis units connected alternatingly and sequentially as described above.

Thus, in this system an aqueous feed stream comprising glyoxylic acid and oxalic acid provided to the first crystallization unit can be subjected to a series of consecutive alternating separation and concentration steps, each subsequent separation-concentration cycle increasing the glyoxylic acid concentration such that a product stream comprising purified glyoxylic acid may be withdrawn from the final reverse osmosis unit in the series.

As described above, the crystallization and reverse osmosis units in this system can be any combination of units suitable for the separation of oxalic acid by crystallization and concentration of the resulting solution by reverse osmosis, respectively.

In one embodiment, one or more of the crystallisation units further comprise an outlet for discharging a stream comprising oxalic acid, wherein said stream comprising oxalic acid is fluidly connected with an oxalic acid recycle stream for recirculation to an inlet of an electrochemical cell. Preferably, this electrochemical cell forms part of a system configured for the electrochemical reduction of oxalic acid to glyoxylic acid, wherein the product stream of this electrochemical cell is used as a feed stream for the glyoxylic acid purification process as described herein.

In one embodiment, said system for the electrochemical reduction of oxalic acid to glyoxylic acid is configured for an electrochemical reduction process of oxalic acid (OA) to glyoxylic acid (GA) wherein additional oxalic acid is supplied to the process in concentrated form and wherein the oxalic acid concentration in the catholyte is kept between pre-defined limits throughout the process, resulting in a concentration of glyoxylic acid in the product stream well exceeding 10 wt%.

In one embodiment an integrated process and system for the electrochemical conversion of oxalic acid to glyoxylic and the subsequent purification of glyoxylic acid is provided, wherein oxalic acid is electrochemically reduced to glyoxylic acid, which is subsequently recovered and purified as described herein and wherein non-converted oxalic is separated in one or more crystallization units is recycled as a feed into the electrochemical process.

The invention will now be described in detail with reference to the non-limiting embodiments shown in the Figure. In the following, for the sake of understanding, elements of embodiments are described in operation. However, it will be apparent that the respective elements are arranged to perform the functions being described as performed by them. Further, the subject matter that is presently disclosed is not limited to the embodiments only, but also includes every other combination of features described herein or recited in mutually different dependent claims.

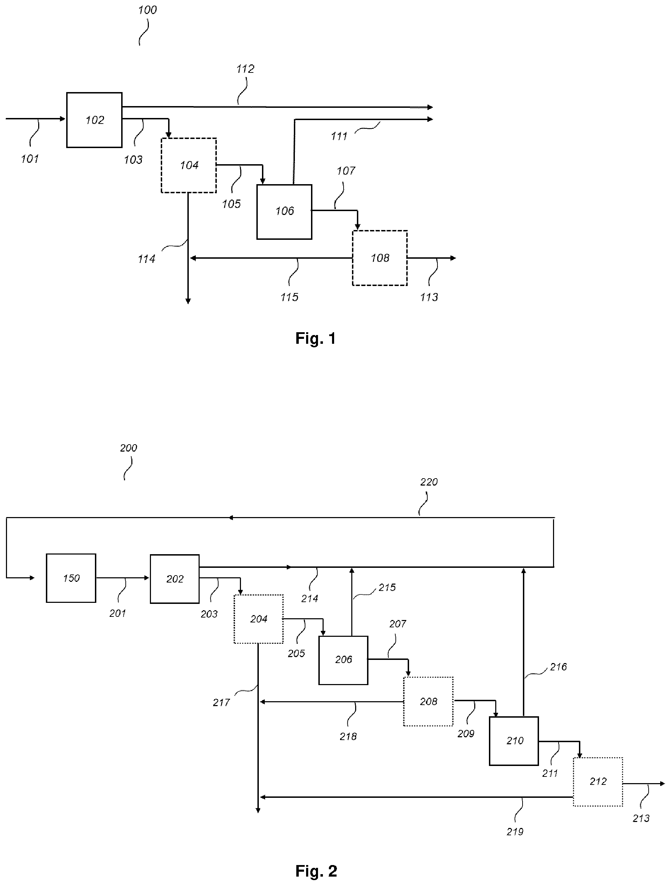

Referring to Figure 1, a schematic illustrating a system 100 for the recovery of glyoxylic acid from an aqueous product stream comprising glyoxylic acid and oxalic acid according to an embodiment of the present disclosure is shown. During operation, a feed stream 101 comprising an aqueous solution comprising oxalic acid and glyoxylic acid is provided to a first crystallization unit 102. In crystallization unit 102, a large portion of the oxalic acid is precipitated by cooling and separated from the solution as oxalic acid stream 112. An aqueous stream 103 depleted in oxalic acid and comprising glyoxylic acid is withdrawn from an outlet of crystallization unit 102 and fed to an inlet of first reverse osmosis unit 104. In reverse osmosis unit 104, water is separated from the glyoxylic acid and any remaining oxalic acid. A water stream 114 is withdrawn from the reverse osmosis unit 104. This water stream 104 may be discarded or used in associated processes, such as for dissolving reactants or for producing steam. A concentrated aqueous stream 105 comprising glyoxylic acid and a minor amount of oxalic acid is withdrawn from an outlet of reverse osmosis unit 104 and fed to an inlet of a second crystallization unit 106, wherein again a large portion of the oxalic acid is precipitated and removed as oxalic acid stream 111 from the solution. A concentrated stream 107 depleted in oxalic acid and comprising glyoxylic acid is withdrawn from an outlet of crystallization unit 106 and fed to an inlet of a second reverse osmosis unit 108. In reverse osmosis unit 108, water is separated from the glyoxylic acid and any optional remaining oxalic acid. A further concentrated stream 113 comprising glyoxylic acid and optionally oxalic acid is withdrawn from an outlet of reverse osmosis unit 108. A water stream 115 is withdrawn from the reverse osmosis unit 108 and discarded or utilized as described above. Optionally, oxalic acid streams 111 and 112 can be used as feed or recycle stream in a system configured for converting, e.g. electrochemically, oxalic acid to organic products.

Referring to Figure 2, a schematic illustrating a system 200 for the recovery of glyoxylic acid from an aqueous product stream comprising glyoxylic acid and oxalic acid according to an embodiment of the present disclosure is shown. During operation, an aqueous product stream comprising an aqueous solution comprising oxalic acid and glyoxylic acid is withdrawn from a electrochemical reaction system 150 and provided as a feed stream 201 to a first crystallization unit 202. In crystallization unit 202, a large portion of the oxalic acid is precipitated by cooling and separated from the solution as oxalic acid stream 212. An aqueous stream 203 depleted in oxalic acid and comprising glyoxylic acid is withdrawn from an outlet of crystallization unit 202 and fed to an inlet of first reverse osmosis unit 204. In reverse osmosis unit 204, water is separated from the glyoxylic acid and any remaining oxalic acid. A water stream 217 is withdrawn from the reverse osmosis unit 204 and discarded or utilized as described above. A concentrated aqueous stream 205 comprising glyoxylic acid and a minor amount of oxalic acid is withdrawn from an outlet of reverse osmosis unit 204 and fed to an inlet of a second crystallization unit 206, wherein again a large portion of the oxalic acid is precipitated and removed as oxalic acid stream 215 from the solution. A concentrated stream 207 depleted in oxalic acid and comprising glyoxylic acid is withdrawn from an outlet of crystallization unit 206 and fed to an inlet of a second reverse osmosis unit 208. In reverse osmosis unit 208, water is separated from the glyoxylic acid and any optional remaining oxalic acid. A water stream 218 is withdrawn from the reverse osmosis unit 208 and discarded or utilized as described above. A further concentrated stream 209 comprising glyoxylic acid and optionally oxalic acid is withdrawn from an outlet of reverse osmosis unit 208 and fed to an inlet of a third crystallization unit 210, wherein again a large portion of the oxalic acid is precipitated and removed as oxalic acid stream 216 from the solution. A stream 211 further depleted in oxalic acid and comprising concentrated glyoxylic acid is withdrawn from an outlet of crystallization unit 210 and fed to an inlet of a third reverse osmosis unit 212. In reverse osmosis unit 212, water is separated from the glyoxylic acid. A further concentrated stream 213 comprising glyoxylic acid is withdrawn from an outlet of reverse osmosis unit 212. A water stream 219 is withdrawn from the reverse osmosis unit 208 and discarded or utilized as described above.

Optionally, oxalic acid streams 214, 215 and/or 216 are recycled as a feed stream to electrochemical reaction system 150.

### Examples

### Example 1: low-temperature crystallization of oxalic acid

Low-temperature crystallization experiments have been carried out on a series of solutions containing glyoxylic acid and oxalic acid at different concentrations.

Solutions containing glyoxylic acid and oxalic acid at different concentrations have been prepared and were cooled under controlled conditions in a Crystal16^{™} parallel crystallizer (Technobis Crystallization Systems). After reaching the set temperature the mixture as left to equilibrate over night after which a sample of the liquid phase was taken to determine the concentration of oxalic acid and glyoxylic acid after crystallization.

The results are collected in Table 1.

| Entry | T setpoint (degree Celsius) | Glyoxylic concentration at RT (gr/L) | Oxalic concentration at RT (gr/L) | Glyoxylic concentration at setpoint (gr/L) | Oxalic concentration at setpoint (gr/L) |
|---|---|---|---|---|---|
| 1 | 10 | 150.4 | 58.8 | 156.1 | 36.3 |
| 2 | 5 | 150.4 | 58.8 | 160.62 | 32.70 |
| 3 | 0 | 150.4 | 58.8 | 157.73 | 27.04 |
| 4 | -5 | 150.4 | 58.8 | 161.70 | 22.37 |
| 5 | 10 | 310.6 | 44.1 | 326.42 | 29.07 |
| 6 | 5 | 310.6 | 44.1 | 323.65 | 21.83 |
| 7 | 0 | 310.6 | 44.1 | 332.54 | 19.25 |
| 8 | -5 | 566.8 | 29.5 | 573.48 | 9.57 |

### Example 2: concentration of glyoxylic acid and oxalic acid

A flat sheet RO membrane is positioned in a Sepa Cell lab test unit. A feed stream comprising 15 wt% glyoxylic acid and 6 wt% oxalic acid is separated over the membrane into a permeate stream and a retentate stream.

To generate permeate flow through the membrane, a feed pressure is applied by means of a positive displacement pump and by adjusting the valve on the concentrate stream. The feed pressure can be adjusted in the range of 10-100 bars.

Furthermore, the temperature of the feed stream is monitored by a thermometer and controlled by a heating plate and cooling spiral element. The feed stream has a temperature in the range of 10-40 °C.

The resulting permeate stream mainly consists of water, whereas the retentate stream consists of a concentrated glyoxylic acid and oxalic acid stream. The flux of permeate through the membrane is monitored at regular intervals by a flow meter. Based on the available active area of the membrane coupon, the flux can be calculated.

## Claims

1. A process for the recovery of glyoxylic acid from an aqueous solution comprising glyoxylic acid and oxalic acid, said process comprising the steps of:
(i) providing an aqueous solution comprising glyoxylic acid and oxalic acid,
(ii) precipitating at least a portion of the oxalic acid using low-temperature crystallization at a temperature of at most 18 °C,
(iii) separating the precipitated oxalic acid from the solution to obtain a solution depleted in oxalic acid,
(iv) concentrating the solution depleted in oxalic obtained in step (iii) using reverse osmosis,
repeating steps (ii)-(iv) with the concentrated solution obtained in step (iv) until a purified glyoxylic acid stream with a predetermined degree of purity is obtained.

2. The process according to claim 1, wherein the aqueous solution of step (i) comprises 5-20 wt%, preferably 8-18 wt%, most preferably 11-13 wt% glyoxylic acid.

3. The process according to any of the preceding claims, wherein the aqueous solution of step (i) comprises 3-12 wt%, preferably 5-10 wt%, more preferably 6-8 wt% oxalic acid.

4. The process according to any of the preceding claims, wherein the number of consecutive precipitation-separation-concentration cycles is at least two, preferably at least three, more preferably at least four.

5. The process according to any of the preceding claims, wherein the temperature in the low-temperature crystallization of step (ii) is in the range of -10 to 15 °C, preferably in the range of -8 to 10 °C, more preferably in the range of -5 to 5 °C.

6. The process of according to any of the preceding claims, wherein the purified glyoxylic acid stream after the final concentration step comprises glyoxylic acid in a concentration at least 40 wt%, more preferably at least 45 wt%, most preferably around 50 wt%.

7. The process according to claim 6, wherein the purified glyoxylic acid stream after the final concentration step is further purified to obtain glyoxylic acid monohydrate with a degree of purity of at least 90 wt%, more preferably at least 95 wt%, most preferably at least 99 wt%.

8. The process according to claim 7, wherein the purified glyoxylic acid stream after the final concentration step is further purified using vacuum distillation.

9. The process according to any of the preceding claims, wherein at least a portion the oxalic acid separated in step (iii) is provided to an electrochemical process for the conversion of oxalic acid to glyoxylic acid.

10. A reaction system for carrying out the process according to any one of the preceding claims, said system comprising a plurality of sequentially and alternatingly connected crystallisation units and reverse osmosis units, wherein an outlet of each crystallisation unit is fluidly connected with an inlet of a reverse osmosis unit following it in sequential order, and wherein the first crystallisation unit is configured to receive an aqueous feed stream comprising glyoxylic acid and oxalic acid, and wherein each subsequent crystallisation unit comprises an inlet fluidly connected with an outlet of a reverse osmosis unit preceding it in sequential order.

11. The reaction system according to claim 10, wherein the system comprises at least two, preferably at least three, more preferably at least four crystallisation units and at least two, preferably at least three, more preferably at least four reverse osmosis units, wherein said crystallisation units and reverse osmosis units are connected alternatingly and sequentially.

12. The reaction system according to claim 10 or 11, wherein one or more of the crystallisation units further comprise an outlet for discharging a stream comprising oxalic acid, said stream being fluidly connected with an oxalic acid recycle stream for recirculation to an inlet of an electrochemical cell configured for the electrochemical conversion of oxalic acid to glyoxylic acid.

## Patentansprüche

1. Verfahren zur Gewinnung von Glyoxylsäure aus einer wässrigen Lösung, die Glyoxylsäure und Oxalsäure umfasst, wobei das Verfahren die Schritte umfasst:
(i) Bereitstellen einer wässrigen Lösung, umfassend Glyoxylsäure und Oxalsäure,
(ii) Ausfällen zumindest eines Teils der Oxalsäure mittels Niedertemperaturkristallisation bei einer Temperatur von höchstens 18 °C,
(iii) Abtrennen der ausgefällten Oxalsäure von der Lösung, um eine an Oxalsäure verarmte Lösung zu erhalten,
(iv) Konzentrieren der in Schritt (iii) erhaltenen an Oxalsäure verarmten Lösung mittels Umkehrosmose,
Wiederholen der Schritte (ii)-(iv) mit der in Schritt (iv) erhaltenen konzentrierten Lösung, bis ein gereinigter Glyoxylsäurestrom mit einem vorbestimmten Reinheitsgrad erhalten wird.

2. Verfahren nach Anspruch 1, wobei die wässrige Lösung von Schritt (i) 5-20 Gew.-%, vorzugsweise 8-18 Gew.-%, am meisten bevorzugt 11-13 Gew.-% Glyoxylsäure umfasst.

3. Verfahren nach einem der vorhergehenden Ansprüche, wobei die wässrige Lösung von Schritt (i) 3-12 Gew.-%, vorzugsweise 5-10 Gew.-%, mehr bevorzugt 6-8 Gew.-% Oxalsäure umfasst.

4. Verfahren nach einem der vorhergehenden Ansprüche, wobei die Anzahl der aufeinanderfolgenden Fällungs-Abtrennungs-Konzentrierungs-Zyklen mindestens zwei, vorzugsweise mindestens drei, mehr bevorzugt mindestens vier beträgt.

5. Verfahren nach einem der vorhergehenden Ansprüche, wobei die Temperatur bei der Niedertemperaturkristallisation von Schritt (ii) im Bereich von -10 bis 15 °C, vorzugsweise im Bereich von -8 bis 10 °C, mehr bevorzugt im Bereich von -5 bis 5 °C liegt.

6. Verfahren nach einem der vorhergehenden Ansprüche, wobei der gereinigte Glyoxylsäurestrom nach dem letzten Konzentrierungsschritt Glyoxylsäure in einer Konzentration von mindestens 40 Gew.-%, mehr bevorzugt 45 Gew.-%, am meisten bevorzugt etwa 50 Gew.-% umfasst.

7. Verfahren nach Anspruch 6, wobei der gereinigte Glyoxylsäurestrom nach dem letzten Konzentrationsschritt weiter gereinigt wird, um Glyoxylsäure-Monohydrat mit einem Reinheitsgrad von mindestens 90 Gew.-%, mehr bevorzug mindestens 95 Gew.-%, am meisten bevorzugt mindestens 99 Gew.-% zu erhalten.

8. Verfahren nach Anspruch 7, wobei der gereinigte Glyoxylsäurestrom nach dem letzten Konzentrationsschritt mittels Vakuumdestillation weiter gereinigt wird.

9. Verfahren nach einem der vorhergehenden Ansprüche, wobei zumindest ein Teil der in Schritt (iii) abgetrennten Oxalsäure einem elektrochemischen Verfahren zur Umwandlung von Oxalsäure in Glyoxylsäure bereitgestellt wird.

10. Reaktionssystem zur Durchführung des Verfahrens nach einem der vorhergehenden Ansprüche, wobei das System eine Vielzahl von sequentiell und abwechselnd verbundenen Kristallisationseinheiten und Umkehrosmoseeinheiten umfasst, wobei ein Auslass jeder Kristallisationseinheit mit einem Einlass einer Umkehrosmoseeinheit, die ihr in sequentieller Reihenfolge folgt, in Fluidverbindung steht und wobei die erste Kristallisationseinheit so konfiguriert ist, dass sie einen wässrigen Zufuhrstrom aufnimmt, der Glyoxylsäure und Oxalsäure umfasst, und wobei jede nachfolgende Kristallisationseinheit einen Einlass umfasst, der in Fluidverbindung mit einem Auslass einer Umkehrosmoseeinheit steht, die ihr in sequentieller Reihenfolge vorausgeht.

11. Reaktionssystem nach Anspruch 10, wobei das System mindestens zwei, vorzugsweise mindestens drei, mehr bevorzugt mindestens vier Kristallisationseinheiten und mindestens zwei, vorzugsweise mindestens drei, mehr bevorzugt mindestens vier Umkehrosmoseeinheiten umfasst, wobei die Kristallisationseinheiten und die Umkehrosmoseeinheiten abwechselnd und in sequentieller Reihenfolge verbunden sind.

12. Reaktionssystem nach Anspruch 10 oder 11, wobei eine oder mehrere der Kristallisationseinheiten ferner einen Auslass zum Ablassen eines Oxalsäure umfassenden Stroms umfassen, wobei der Strom in Fluidverbindung mit einem Oxalsäure-Rückführstrom zur Rückführung zu einem Einlass einer elektrochemischen Zelle steht, die für die elektrochemische Umwandlung von Oxalsäure in Glyoxylsäure konfiguriert ist.

## Revendications

1. Procédé de récupération d'acide glyoxylique à partir d'une solution aqueuse comprenant de l'acide glyoxylique et de l'acide oxalique, ledit procédé comprenant les étapes consistant à :
(i) la fourniture d'une solution aqueuse comprenant de l'acide glyoxylique et de l'acide oxalique,
(ii) la précipitation d'au moins une partie de l'acide oxalique à l'aide de la cristallisation à basse température à une température maximale de 18 °C,
(iii) la séparation de l'acide oxalique précipité de la solution pour obtenir une solution appauvrie en acide oxalique,
(iv) la concentration de la solution appauvrie en oxalique obtenue à l'étape (iii) à l'aide de l'osmose inverse,
la répétition des étapes (ii)-(iv) avec la solution concentrée obtenue à l'étape (iv) jusqu'à l'obtention d'un courant d'acide glyoxylique purifié avec un degré de pureté prédéterminé.

2. Procédé selon la revendication 1, dans lequel la solution aqueuse de l'étape (i) comprend 5 à 20 % en poids, de préférence 8 à 18 % en poids, le plus préférablement 11 à 13 % en poids d'acide glyoxylique.

3. Procédé selon l'une quelconque des revendications précédentes, dans lequel la solution aqueuse de l'étape (i) comprend 3 à 12 % en poids, de préférence 5 à 10 % en poids, plus préférablement 6 à 8 % en poids d'acide oxalique.

4. Procédé selon l'une quelconque des revendications précédentes, dans lequel le nombre de cycles consécutifs de précipitation-séparation-concentration est d'au moins deux, de préférence d'au moins trois, plus préférablement d'au moins quatre.

5. Procédé selon l'une quelconque des revendications précédentes, dans lequel la température de la cristallisation à basse température de l'étape (ii) est dans la plage comprise entre -10 et 15 °C, de préférence entre -8 et 10 °C, plus préférablement entre -5 et 5 °C.

6. Procédé selon l'une quelconque des revendications précédentes, dans lequel le flux d'acide glyoxylique purifié après l'étape de concentration finale comprend de l'acide glyoxylique à une concentration d'au moins 40 % en poids, plus préférablement d'au moins 45 % en poids, le plus préférablement d'environ 50 % en poids.

7. Procédé selon la revendication 6, dans lequel le flux d'acide glyoxylique purifié après l'étape de concentration finale est en outre purifié pour obtenir de l'acide glyoxylique monohydraté avec un degré de pureté d'au moins 90 % en poids, plus préférablement d'au moins 95 % en poids, le plus préférablement d'au moins 99 % en poids.

8. Procédé selon la revendication 7, dans lequel le flux d'acide glyoxylique purifié après l'étape de concentration finale est en outre purifié à l'aide de la distillation sous vide.

9. Procédé selon l'une quelconque des revendications précédentes, dans lequel au moins une partie de l'acide oxalique séparé à l'étape (iii) est fournie à un procédé électrochimique pour la conversion de l'acide oxalique en acide glyoxylique.

10. Système de réaction pour la mise en oeuvre du procédé selon l'une quelconque des revendications précédentes, ledit système comprenant une pluralité d'unités de cristallisation et d'unités d'osmose inverse séquentiellement et alternativement reliées, dans lequel une sortie de chaque unité de cristallisation est fluidiquement reliée à une entrée d'une unité d'osmose inversequi la suit dans l'ordre séquentiel, et dans lequel la première unité de cristallisation est conçue pour recevoir un flux d'alimentation aqueux comprenant de l'acide glyoxylique et de l'acide oxalique, et dans lequel chaque unité de cristallisation suivante comprend une entrée reliée fluidiquement à une sortie d'une unité d'osmose inverse qui la précède dans l'ordre séquentiel.

11. Système de réaction selon la revendication 10, dans lequel le système comprend au moins deux, de préférence au moins trois, plus préférablement au moins quatre unités de cristallisation et au moins deux, de préférence au moins trois, plus préférablement au moins quatre unités d'osmose inverse, dans lequel lesdites unités de cristallisation et d'osmose inverse sont reliées alternativement et séquentiellement.

12. Système de réaction selon la revendication 10 ou 11, dans lequel une ou plusieurs des unités de cristallisation comprennent en outre une sortie pour décharger un flux comprenant de l'acide oxalique, ledit flux étant relié fluidiquement à un flux de recyclage de l'acide oxalique pour la recirculation vers une entrée d'une cellule électrochimique conçue pour la conversion électrochimique de l'acide oxalique en acide glyoxylique.
